# EUROPEAN PATENT APPLICATION

(11) **EP 1 203 584 A1**
(43) Date of publication of application: **08.05.2002**
(21) Application number: 01123726.0
(22) Date of filing: 04.10.2001
(51) Int. Cl.: A61K 31/27, A61K 31/66, A61P 25/28

(54) **Cholinergic precursor (in particular choline alfoscerate) associated with an acetylcholinesterase inhibitor (such as rivastigmine, donepezil)**

(30) Priority: 13.10.2000 IT PD000238; 12.04.2001 IT PD010091
(71) Applicant: M.D.M. S.r.l., 20123 Milano (IT)
(72) Inventor: Trognoni, Mariano, 20123 Milano (IT)
(74) Representative: Gustorf, Gerhard, Dipl.-Ing.

(57) **Abstract**

The invention is a new combined drug, particularly effective in treating cognitive disorders specific to adults, including Alzheimer's disease, consisting in the combination of a cholinergic precursor (e.g. choline alfoscerate) and an AChE inhibitor (e.g. rivastigmine). The combination permits the use of lower doses of AChE inhibitors, resulting in a reduction in the incidence of side and/or toxic effects induced by long-term treatment with AchE inhibitors. Furthermore, the new drug makes higher quantities of choline, and therefore acetylcholine, available in patients in which the inhibition of AChE only may not be effective due to the low neurotransmitter levels present in the cholinergic synapses.

## Description

The present patent concerns the pharmacological sector and in particular it concerns a new combined drug which is particularly effective in treating cognitive disorders specific to adults, including Alzheimer's disease.

At present drugs exist on the market with the active principles necessary for the production of acetylcholine.

Acetylcholine is, in fact, the most important neurotransmitter in cognitive processes and its activity can be attributed to a "probabilistic" meeting between a molecule of acetylcholine and a specific post-synaptic receptor.

In physiological conditions, e.g. in a human end plate, 3 million molecules of acetylcholine are discharged into the junction at each individual impulse, thus guaranteeing that the probability of the neurotransmitter-receptor meeting is practically 100%.

The role of the cholinergic products is to increase the availability of choline for the inadequate neuron, therefore helping to re-establish the maximum physiological presence of neurotransmitters within the intersynaptic space.

The role of the anticholinesterasic products is to limit the deterioration of the acetylcholine already formed, thus attempting to achieve the same effect, in other words to increase the concentration of acetylcholine in the intersynaptic space.

In the latter case, however, the existing production (not necessarily sufficient in itself) can be preserved, but without stimulating the process of new production which could be essential for achieving the "critical mass".

It is therefore assumed that the low number of responders *(approximately 30-40%)* to the anticholinesterasic products currently available on the market may be due to this latter aspect.

The non-persistent effectiveness of the responders in the long-term could also be due to the fact that the contribution of choline (in the absence of specific pharmacological intervention) cannot compensate for the normal deterioration of the choline itself and its excretion, with the result that less and less acetylcholine can be preserved via the use of anticholinesterasic products alone (which are undoubtedly useful as drugs but not free from side effects, some of which are significant).

The aim of the present invention is to combine said products to obtain a drug with a therapeutic effect not achieved before, since said combined action of cholinergic and anticholinesterasic drugs is particularly effective in solving all the above-mentioned problems.

It would therefore appear logical to say that cholinergic drugs able to pass the blood-brain barrier (in particular alfa-GFC) make a useful contribution to the action of the anticholinesterasic drugs, both by reducing the number of non-responders and by guaranteeing greater long-term effectiveness of the same.

By combining the drugs, the doses of each individual drug if taken alone can be reduced, therefore obtaining a better result and simultaneously also reducing the side effects of a necessarily continuous treatment.

The present invention concerns a new drug resulting from the combination of a cholinergic precursor (e.g. choline alfoscerate) and an AchE inhibitor (e.g. rivastigmine or similar).

The advantage of the combination consists in the possibility of using smaller doses of the AChE inhibitors necessary to induce a significant increase in the availability of acetylcholine at the level of the intersynaptic space.

This is useful for reducing the incidence of side and/or toxic effects induced by long-term treatment with AchE inhibitors.

Another advantage of the combination is that larger amounts of choline and therefore acetylcholine are made available in patients in which AChE inhibition alone may not be effective due to the low neurotransmitter levels present in the cholinergic synapses.

The effects of the combination of the cholinergic precursor choline alfoscerate and rivastigmine (acetylcholinesterase inhibitor) on the levels of acetylcholine in the cerebral cortex (frontal area), in the hippocampus and in the corpus striatum of rats, and on the immunoreactivity of the neuronal acetylcholine in the neurones of the magnocellular nucleus basalis (MNB) and in the corpus striatum were studied.

Male Wistar rats were divided into 5 groups, one of which was a control group (non-treated), one treated for 1 week by intraperitoneal administration of 150 mg/kg of choline alfoscerate, one treated with a daily oral dose of 2.5 mg/kg of rivastigmine, the fourth with 150 mg/kg of choline alfoscerate plus 0.625 mg/kg of rivastigmine and the fifth with 150 mg/kg of choline alfoscerate plus 2.5 mg/kg of rivastigmine.

The dosage of 150 mg/kg of choline alfoscerate was chosen as it significantly increases the release of acetylcholine both in the hippocampus and in the corpus striatum, while the dose of 2.5 mg/kg of rivastigmine inhibits the activity of the acetylcholinesterase (AChE) by over 50%.

The drugs administered individually (choline alfoscerate or rivastigmine) increased the levels of acetylcholine by approximately 20%. Administration of the two drugs in combination induced a more marked increase in the levels of acetylcholine compared to the individual treatment. Choline alfoscerate combined with the lowest dose of rivastigmine increased the levels of acetylcholine by approximately 30% in the three cerebral areas studied, while the combination of choline alfoscerate with the highest doses of rivastigmine increased the levels of acetylcholine by approximately 35%.

The dosage of 2.5 mg/kg of rivastigmine, alone or combined with choline alfoscerate, reduced the activity of the AChE by approximately 50%, while the dosage of 0.625 mg/kg of rivastigmine reduced the activity of the AChE by approximately 25%.

The choline alfoscerate alone did not show any effect on the activity of the AChE.

In agreement with the findings for the levels of acetylcholine, both the choline alfoscerate and the rivastigmine, administered alone, increased in a similar fashion the immunoreactivity of the acetylcholine in the MNB neurones as in the interneurones of the corpus striatum, with a more marked effect at the level of the MNB.

The combined administration of choline alfoscerate and rivastigmine increased the immunoreactivity of the acetylcholine both in the neurones of the MNB and in the interneurones of the corpus striatum to higher levels than the individual compounds on their own.

Increase in the immunoreactivity was not statistically different using the lowest dose of rivastigmine (0.625 mg/kg) with respect to the highest dose (2.5 mg/kg).

The results described have shown that the choline alfoscerate induces an increase in the levels of acetylcholine in the frontal cerebral cortex, in the hippocampus and in the corpus striatum, to the same extent as the dosage of the AchE inhibitor, rivastigmine, which is able to inhibit said enzyme by approximately 50% (2.5 mg/kg). Combination of the acetylcholine precursor and the AChE inhibitor increased the levels of acetylcholine and neuronal immunoreactivity to a greater extent than the individual compounds administered alone.

The combination of choline alfoscerate and a dose of rivastigmine 4 times lower than the EC₅₀ caused an increase in the levels of acetylcholine and immunoreactivity by approximately 30%, in a more marked fashion in an area correlated with learning and memory such as the hippocampus.

These results demonstrate that the combination of an acetylcholine biosynthesis precursor, like choline alfoscerate, and an AChE inhibitor represents a valid strategy for cholinergic replacement therapy of cognitive disorders specific to adults, including Alzheimer's disease.

The attached figures illustrate (a) application of anticholinesterasic drugs only, and (b) combined application of anticholinesterasic and cholinergic drugs. (A) indicates the nerve axon, (M) the mitochondria, (VS) the synaptic vesicles, (RA) the acetylcholine receptors, (FS) the synaptic cleft, (PS) the sarcolemmal folds and (S) the sarcoplasm.

The following claims are therefore made with reference to the above description.

## Claims

1. Drug for the treatment of Alzheimer's disease, **characterised in that** it comprises and combines cholinergic drugs (e.g, alfa-GFC) with anticholinesterasic drugs such as Rivastigmine, Donepezil, Tacrine and similar.

2. Combined drug for cholinergic replacement therapy of cognitive disorders specific to adults, including Alzheimer's disease, **characterised in that** it comprises the combination of a cholinergic precursor (e.g. choline alfoscerate) and an acetylcholinesterase inhibitor (e.g. rivastigmine and similar).

3. Cholinergic drug (e.g. alfa-GFC) for the treatment of Alzheimer's disease, **characterised in that** it is combined with anticholinesterasic drugs such as Rivastigmine, Donepezil, Tacrine and similar.

4. Anticholinesterasic drug such as Rivastigmine, Donepezil, Tacrine and similar for the treatment of Alzheimer's disease, **characterised in that** it is combined with a cholinergic drug (e.g. alfa-GFC).

5. Drug according to claims 1., 2., 3., 4., useful in the treatment of non-responders to anticholinesterasic drugs only.
